# EUROPEAN PATENT SPECIFICATION

(11) **EP 4 162 855 B1**
(45) Date of publication and mention of the grant of the patent: **06.08.2025**
(21) Application number: 20940752.7
(22) Date of filing: 02.07.2020
(51) Int. Cl.: A47L 11/34, A47L 13/22, A47L 11/30

(54) **SURFACE CLEANING APPARATUS**
OBERFLÄCHENREINIGUNGSVORRICHTUNG
APPAREIL DE NETTOYAGE DE SURFACE

(30) Priority: 18.06.2020 CN 202010559797
(43) Date of publication of application: 12.04.2023
(73) Proprietor: Suzhou Eup Electric Co. Ltd., Suzhou, Jiangsu 215011 (CN)
(72) Inventor: BIAN, Zhuang, Suzhou, Jiangsu 215011 (CN)
(74) Representative: Sun, Yiming
(86) International application number: PCT/CN2020/099897
(87) International publication number: WO 2021/253509

(56) References cited:
- EP-A1- 3 202 299
- CN-A- 1 771 879
- CN-A- 102 240 190
- CN-A- 102 240 190
- CN-A- 110 507 241
- CN-A- 110 811 413
- CN-A- 116 035 483
- CN-U- 207 152 545
- CN-U- 208 435 476
- CN-U- 209 220 154
- US-A- 3 755 850
- US-A- 5 613 271
- US-A- 5 613 271
- US-A1- 2019 290 087

## Description

### TECHNICAL FIELD

The present invention relates to a surface cleaning apparatus, and in particular to a wet surface cleaning apparatus for cleaning by applying a cleaning liquid and steam to a surface to be cleaned and recovering the dirty liquid after cleaning.

### BACKGROUND

It is well known that wet floor cleaning apparatuses are known, for example, in the form of a floor cleaning apparatus or a floor scrubbing apparatus. Some of the apparatuses use one or more cleaning rollers rotating about one or more horizontal axes as cleaning tools; some use a pair of scrubbing trays that rotates about a vertical axis perpendicular to a horizontal plane as cleaning tools; and some use a pair of scrubbing sheets that is configured to reciprocally move in a front-rear direction or a left-right direction as cleaning tools. These cleaning tools contact with floors to be cleaned and clean them with the aid of a cleaning liquid. The cleaning liquid is stored in a cleaning liquid supply container and applied to the floors during the cleaning process. Subsequently, some apparatuses suck the applied cleaning liquid together with cleaned dirt (which may include dirt, dust, stain, mud, hair and other debris) from the floors under the action of the cleaning roller and a suction unit and convey them to a dirty liquid storage container; and some apparatuses only recover the dirt on the surface to be cleaned. This recovery may be performed by using a suction source for suction, or the dirty liquid and/or dirt may be recovered in a manner similar to that of a sweeper.

Most of the aforementioned floor cleaning apparatuses have the following problems: during the cleaning process, the liquid applied to the floor is not recovered completely; there is residual dirty liquid on the cleaned floor; and a user has to wait for a long period of time (about 15 minutes) before he or she walks on the cleaned floor so that the dirty liquid on the floor may evaporate naturally.

In order to solve the aforementioned problems, in the prior art, it is proposed to add an absorbent cloth or a dry cleaning roller to the back side of the cleaning roller. Such a solution may solve the problem of the residual dirty liquid on the floor to a certain extent but, at the same time, bring another problem: after the absorbent cloth or the dry cleaning roller is used, the user has to manually disassemble and clean or dry it, which increases the workload of the user and leads to poor user experience.

Therefore, although great progress has been in all aspects of the wet surface cleaning apparatus, consumers still expect to improve the surface cleaning apparatus, so that the time spent in waiting for the surface to dry after the wet cleaning is completed is shortened and not too much or no excessive workload is increased, thereby improving user experience.

A surface cleaning apparatus according to the preamble of claim 1 is disclosed in document CN 208 435 476 U.

### SUMMARY

In view of the above technical problems, the purpose of the present invention is to improve the above-mentioned generic surface cleaning apparatus to provide a surface cleaning equipment that has both shortened waiting time and no extra workload.

In order to achieve the object of the invention,, the present invention provides a surface cleaning apparatus, comprising: a suction source; a cleaning head adapted to move along a surface to be cleaned, the head including a housing and a cleaning roller, the housing provided with a roller chamber having an opening in a lower portion of the housing and a front suction mouth in fluid communication with the roller chamber, the roller positioned in the roller chamber in such a way as to be able to rotate, at least a portion of the cleaning roller extending through the opening configured to contact the surface to be cleaned, and the front suction mouth in fluid communication with the suction source; a cleaning liquid supply tank adapted to store cleaning liquid; a steam generator in fluid communication with the supply tank and configured to heat liquid to generate steam; a first fluid delivery unit including at least one cleaning liquid outlet for applying the cleaning liquid in the cleaning liquid supply tank to the cleaning roller and/or the surface to be cleaned; a second fluid delivery unit including at least one steam outlet for applying the steam generated by the steam generator to the surface to be cleaned; and a dirty liquid recovery tank configured to accept dirty liquid and dirt, the dirty liquid recovery tank being in fluid communication with the front suction mouth; wherein the at least one steam outlet is arranged at the lower portion of the housing and configured to heat the surface by overflowing the steam to the surface to accelerate the evaporation of residual cleaning liquid on the surface.

The at least one steam outlet is preferably configured to spray the steam directly to the surface to be cleaned.

Preferably, the at least one steam outlet is at least partially positioned behind the opening to enable the surface cleaning apparatus to heat a surface cleaned by the cleaning roller with the steam overflowing from the at least one steam outlet.

The cleaning head preferably includes a flexible partition arranged between the at least one steam outlet and the opening.

The cleaning head preferably includes a rear suction mouth provided at a rear portion of the housing, the rear suction mouth is positioned behind the at least one steam outlet, and the rear suction mouth is configured to be in fluid communication with the suction source. Further preferably, the cleaning head includes a flexible flat suction nozzle arranged at the lower portion of the housing, wherein the flexible flat suction nozzle comprises a pair of flexible scraper lips arranged relatively front and back to each other , and the rear suction mouth is formed at the flexible flat suction nozzle; and at least one of the flexible scraper lips interferes with the surface to be cleaned when the cleaning head moves along the surface.

The cleaning head preferably includes a flexible liquid-scraping strip positioned behind the at least one steam outlet, and the strip interferes with the surface to be cleaned when the cleaning head moves along the surface to be cleaned.

The at least one steam outlet is preferably plural in number.

The cleaning head preferably includes a steam diffuser for diffusing steam generated by the steam generator is provided at the lower portion of the housing, and the plurality of steam outlets are located at a lower portion of the steam diffuser.

In a preferred embodiment, the steam diffuser comprises a steam bottom plate, and the plurality of steam outlets are arranged on a lower surface of the steam bottom plate.

The surface cleaning apparatus preferably comprises a body, the body having a handle on the top, and the cleaning head is rotationally coupled to a lower portion of the body. The dirty liquid recovery tank and the cleaning liquid supply tank are preferably both detachably positioned on the body.

The first fluid delivery unit preferably comprises a first pump arranged between the cleaning liquid supply tank and the at least one cleaning liquid outlet.

The second fluid delivery unit preferably comprises a second pump arranged between the cleaning liquid supply tank and the steam generator.

The cleaning head preferably includes a cleaning cloth for scrubbing the surface to be cleaned is provided at the lower portion of the housing, the cleaning cloth is made of a steam-permeable material, and the steam outlet is configured to spray the steam to the cleaning cloth.

The cleaning head preferably includes a brush for scrubbing the surface to be cleaned is further provided at the lower portion of the housing.

In a preferred embodiment, , the at least one steam outlet is at least partially arranged on the front side or left and right sides of the opening.

The steam is introduced on the basis of wet cleaning with the cleaning liquid in the surface cleaning apparatus of the present invention, and the surface is heated by the steam. It can accelerate the evaporation of the residual liquid on the surface, thereby shorten the time the user waits for the surface to dry; and because of the high temperature of steam, it can also be high temperature sterilization of the surface when in contact with the surface, so as to achieve a deeper level of cleaning of the surface.

### BRIEF DESCRIPTION OF THE DRAWINGS

Figure 1 is stereogram I of a surface cleaning apparatus according to an embodiment of the present invention;
Figure 2 is stereogram II of a surface cleaning apparatus according to an embodiment of the present invention, wherein a portion of a rear of a housing is removed;
Figure 3 is a schematic diagram of an internal structure of a cleaning head according to an embodiment of the present invention;
Figure 4 is a stereogram of a steam bottom plate according to an embodiment of the present invention;
Figure 5 is an enlarged schematic diagram of Fig. 3 at A;
Figure 6 is a schematic diagram of the working principle of the surface cleaning apparatus according to an embodiment of the present invention..

### DESCRIPTION OF EMBODIMENTS

In order to describe in detail the technical content, structural features, achieved objectives and effects of the invention, the present invention will be described below in detail through embodiments in combination with accompanying drawings.

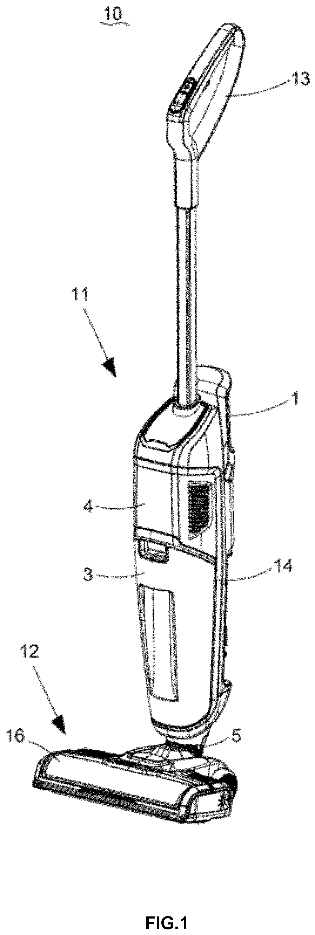

FIG. 1 shows a schematic diagram of a surface cleaning apparatus 10 according to a first embodiment of the present invention. The surface cleaning apparatus 10 is a vertical surface cleaning apparatus and includes a body 11 and a cleaning head 12. A lower portion of the body 11 is articulately connected to the cleaning head 12, and the body 11 can be movable between an upright parked position and a backwardly reclined using position relative to the cleaning head 12. During the cleaning process of the surface cleaning apparatus 10 along the surface to be cleaned, the cleaning head 12 is supported on the surface to be cleaned and guided by the body 11 to move along the surface to be cleaned. The apparatus can be held upright after being used.

For the purpose of description related to the drawings, positional relationships defined by the terms "upper", "lower", "left", "right", "front", "rear", "vertical", "horizontal", "internal", "external" and their derivatives are determined on the basis that a user stands on a surface to be cleaned and at a rear side of a surface cleaning apparatus. When being used, the surface cleaning apparatus of the present invention is pushed from back to front to move forwards or pulled from front to back to move backwards. As used herein, the term "rear side" refers to a position behind at least another component, but does not necessarily mean behind all other components, and the same applies to terms "front side" and "upper side"; and the term "lower portion" refers to a position at or near the bottom of a component. However, it should be understood that various alternative orientations may be used in the present invention unless the opposite orientation is explicitly indicated.

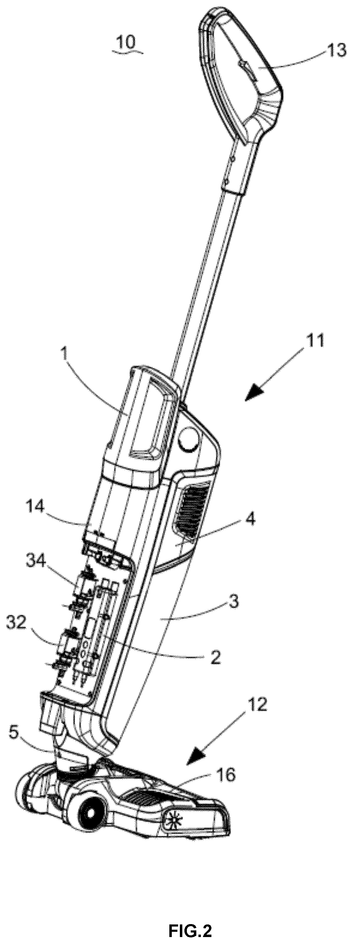

As shown in FIGS. 1 and 2, a cleaning liquid supply system, a steam supply system, and a dirty liquid recovery system are arranged on the surface cleaning apparatus 10. The cleaning liquid supply system is used to store a supply of cleaning liquid and deliver the cleaning liquid to the cleaning head, and the cleaning liquid supply system includes a cleaning liquid supply tank 1 which is used to store the cleaning liquid. The steam supply system is used to heat and convert the cleaning liquid into high-temperature steam by a steam generator 2 and distribute the steam to the surface to be cleaned. The dirty liquid recovery system is used to remove the dirty liquid converted by the used cleaning liquid and dirt on the surface to be cleaned from the surface to be cleaned, and store the dirty liquid and dirt within a certain amount of time. The dirty liquid recovery system includes a dirty liquid recovery tank 3 for accepting dirty liquid and dirt for a certain period of time and a suction source 4.

A handle 13 for the user to hold is mounted on the top of the body 11. The operator may use the handle 13 to hold the surface cleaning apparatus 10 with one hand and then push the cleaning head 12 of the surface cleaning apparatus 10 to move frontwards and backwards on the surface to be cleaned when the surface cleaning apparatus is at the backwardly reclined using position. In other embodiments, the handle can also include a user interface assembly consisting of one or more operating elements. The elements can be such as but not limited to buttons, flip-flops, triggers, switches or the like, operably connected to systems in the apparatus to affect and control function. The operation of all controlled components in the surface cleaning apparatus can be controlled with these operating elements, for example, the surface cleaning apparatus is switched on or off for the cleaning operation, and the delivering of the cleaning fluid and the power of a suction apparatus are adjusted. In other embodiments, the user interface assembly can also be provided in other places of the surface cleaning apparatus, such as on a top wall or side wall of the body under the handle.

The body 11 further comprises a housing 14. The housing 14 is a main supporting part of the body 11 and mainly plays a role of accommodating or carrying the rest of the components on the body 11, such as carrying the suction source 4, the handle 13, the cleaning liquid supply tank 1, the steam generator 2, the dirty liquid recovery tank 3 and the like.

The suction source 4 is a suction force forming component of the dirty liquid recovery system and is used for generating a working airflow. The suction source 4 of the present embodiment comprises a suction motor which is arranged in the housing 14 and constituted by a motor/ fan assembly. The suction motor is capable of providing a fluid flow force extending to the cleaning head.

The cleaning liquid supply tank 1 comprises at least one chamber for holding the cleaning liquid. In other embodiments, the cleaning liquid supply tank can comprise a plurality of chambers. In the present embodiment, the cleaning liquid is water. In other embodiments, the cleaning liquid may be other types of cleaning liquids. The cleaning liquid supply tank 1 is detachably mounted on the housing 14. The user can replenish the cleaning liquid supply tank 1 with the cleaning liquid at any time as required.

The dirty liquid recovery tank 3 is detachably mounted on a front portion of the housing 14. With such a structure, the user can conveniently empty the dirty liquid recovery tank 3 at any time as required. In other embodiments, the dirty liquid recovery tank can be mounted at other locations, even anywhere on the housing 14.

The steam generator 2 is a steam conversion component which is capable of heating the cleaning liquid from the cleaning liquid supply tank 1 to at least 100°C, and convert it into high-temperature steam output.

The cleaning head 12 comprises a housing 16 which is pivotally mounted the lower portion of the body 11 via a rotating joint 5. The rotating joint 5 enables the body 11 to rotate between the upright parked position and the backwardly reclined using position.

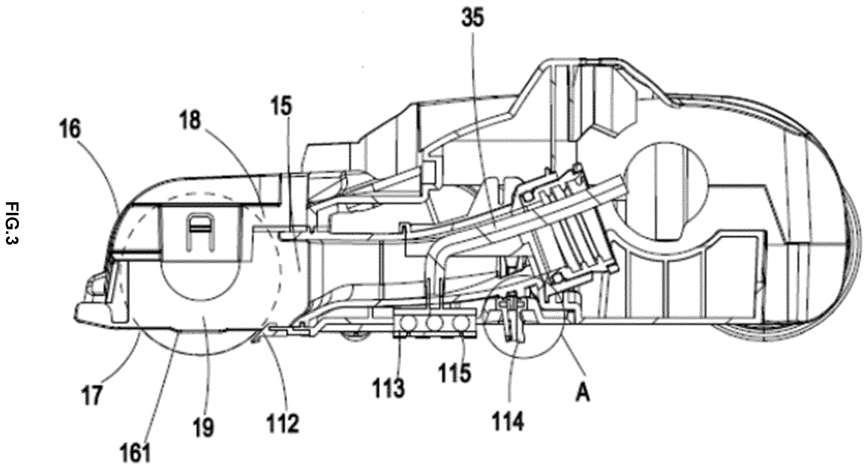

FIG. 3 shows and describes an internal structure of the cleaning head 12. A roller chamber 18 with an opening 17 is defined on the housing 16, a cleaning roller 19 is positioned in the roller chamber 18 in such a way as to be able to rotate, and a driving arrangement (not shown in the figure) which drives the cleaning roller 19 to rotate is arranged inside the housing 16. In the present embodiment, the opening 17 is formed in a lower portion 161 of the housing 16, and a lower portion of the cleaning roller 19 protrudes downwards from the opening 17 to contact the surface to be cleaned. A front suction mouth 15 is arranged in the housing 16 and communicates with both the roller chamber 18 and the suction source 4.

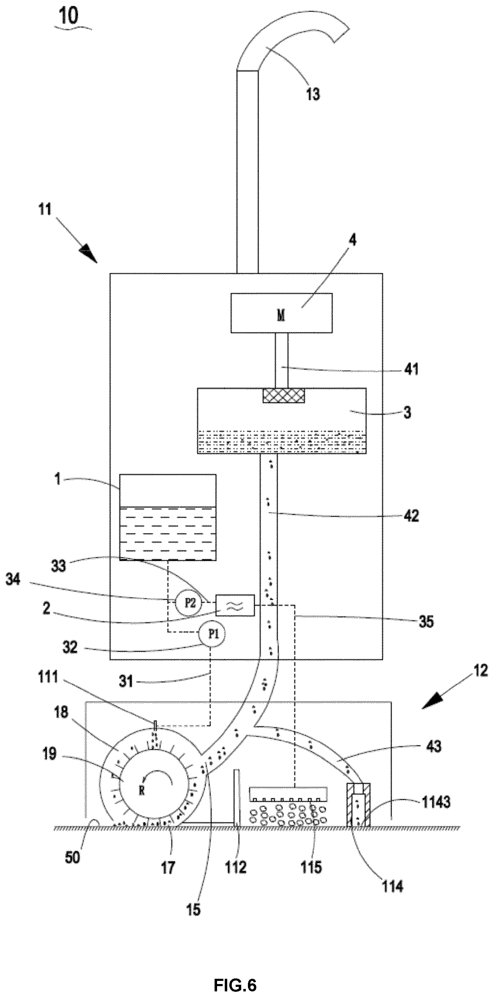

With reference to FIG. 6, the cleaning head 12 is also provided with, inside the roller chamber 18, a cleaning liquid outlet 111 located around the cleaning roller 19, and the cleaning liquid outlet 111 communicates with the cleaning liquid supply tank 1 and can deliver the cleaning liquid in the cleaning liquid supply tank 1 to the cleaning roller 19. In other embodiments, the cleaning liquid outlet may also be arranged to deliver the cleaning liquid toward the surface to be cleaned without the aid of the cleaning roller, but to directly apply the cleaning liquid directly to the surface to be cleaned or to simultaneously deliver the cleaning liquid to both the cleaning roller and the surface to be cleaned. For example, the cleaning liquid outlet may be positioned in a front side of the cleaning roller.

In the present embodiment, an outside surface of the cleaning roller 19 is covered with cleaning materials such as bristles, fabrics, or the similar, and these cleaning materials are configured to be wetted by the cleaning liquid such as water.

As shown in FIG. 3 again, the lower portion 161 of the housing 16 is further provided with a flexible partition 112, a steam bottom plate 113 and a flexible flat suction nozzle 114 from front to back in sequence at the rear side of the opening 17. The flexible flat suction nozzle 114 is located behind the steam outlet 115 and communicates with the suction source 4.

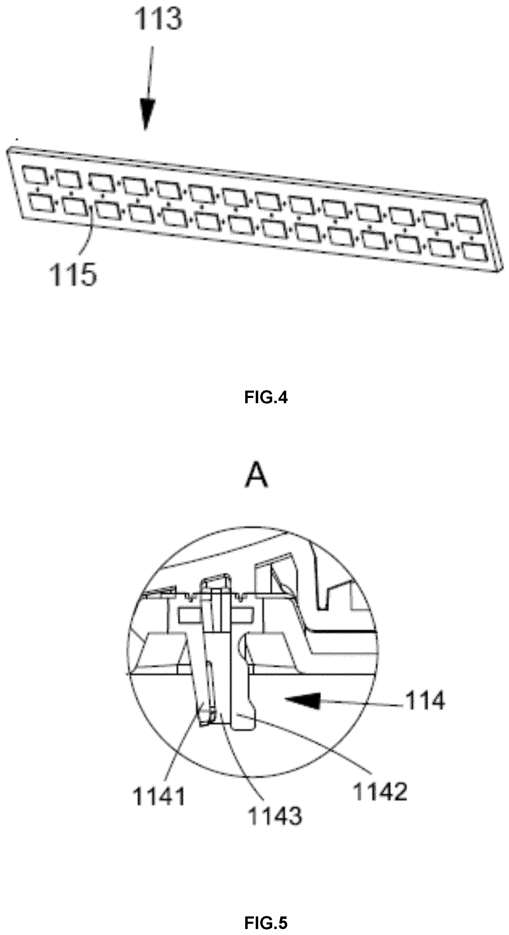

As shown in FIG. 4, the steam bottom plate 113 plays a role of diffusing the steam so as to apply the steam to the surface to be cleaned on the lower side of the steam bottom plate 113 as evenly as possible. In the present embodiment, the steam bottom plate 113 is rectangular, and is provided with a plurality of steam outlets 115 on the lower surface thereof. In the present embodiment, the steam bottom plate 113 and the lower portion 161 of the housing 16 are provided in separate members. In other embodiments, both the steam bottom plate 113 and the lower portion 161 can also be formed in one body. The steam outlet 115 can spray high temperature steam generated by the steam generator 2 to the surface to be cleaned to heat the surface to be cleaned. In the present embodiment, the steam outlet 115 is configured to spray high-temperature steam directly to the surface to be cleaned. The outlet opening of the steam outlet 115 is not limited to being perpendicular to the surface to be cleaned and may also be inclined to a certain degree relative to the surface to be cleaned as long as the structure may overflow steam to the surface to be cleaned. In other embodiments, some cleaning components made of steam-permeable materials, such as steam-permeable cleaning cloths and brushes, may also be provided at the steam outlet. The cleaning components should be configured to ensure that the steam cannot be prevented from being delivered to the surface to be cleaned. When the cleaning head is designed, no matter the steam output from the steam outlet is directly applied to the surface to be cleaned or delivered to the surface to be cleaned after passing through the cleaning component made of the permeable material, it must be ensured that the temperature of the steam is still high enough, preferably the temperature reaches 100°C or above, when the steam contacts with the surface to be cleaned.

As shown in FIG. 3 again, the flexible partition 112 is arranged between the opening 17 and the steam bottom plate 113 and may prevent steam from escaping from the steam outlet 115 to the opening 17.

As shown in FIG. 5, the flexible flat suction nozzle 114 is configured to be superior to the structure sucking the liquid and small particles. In the present embodiment, the flexible flat suction nozzle 114 comprises a front lip 1141 and a rear scraper lip 1142 which are relatively front and back to each other, and a rear suction mouth 1143 is formed between the front scraper lip 1141 and the rear scraper lip 1142. The flexible flat suction nozzle 114 can suck the liquid remaining on the surface to be cleaned. The liquid may be water droplets condensed from steam overflowing from the steam outlet 115 and remaining on the surface to be cleaned, or residual liquid not being removed from the cleaned surface after the cleaning roller passes by. Likewise, in order to prevent the rear suction mouth 1143 from directly sucking away the steam escaping from the steam outlet 115, a component similar to the aforementioned "flexible partition 112" should also be provided between the rear suction mouth 1143 and the steam outlet 115.

In other embodiments, the flexible flat suction nozzle and the rear suction mouth may also be cancelled. Instead, a liquid-scraping strip capable of scraping the liquid on the flat surface is used. Under the circumstance that the flexible flat suction nozzle and the rear suction mouth are cancelled, all the liquid on the surface to be cleaned is sucked in through the front suction mouth in the front portion of the housing. In this case, in order to suck all the liquid on the surface to be cleaned, the cleaning head should reciprocate on the surface to be cleaned time and time again.

With reference to FIG. 6, the suction source 4 is in fluid communication with the dirty liquid recovery tank 3 through an upper suction passage 41. The dirty liquid recovery tank 3 communicates with the front suction mouth 15 in the cleaning head 3 through a lower suction passage 42. The rear suction mouth 1143 of the flexible flat suction nozzle 114 communicates with the lower suction passage 42 through a suction bypass 43. A first cleaning liquid output pipeline 31 is arranged between the cleaning liquid supply tank 1 and the cleaning liquid outlet 111, and a pump 32 is arranged on the first cleaning liquid output pipeline 31. The cleaning liquid in the cleaning liquid supply tank 1 may be directly delivered to the cleaning liquid outlet 111 by starting the pump 32. A second cleaning liquid output pipeline 33 is arranged between the cleaning liquid supply tank 1 and the steam generator 2, and a pump 34 is provided in the second cleaning liquid output pipeline 33. The cleaning liquid in the cleaning liquid supply tank 1 can be delivered to the steam generator 2 by starting the pump 34. A steam output pipeline 35 is arranged between an output end of the steam generator 2 and a steam outlet 115, and the cleaning liquid is converted into high-temperature steam in the steam generator 2 and delivered to the steam outlet 115 through the steam output pipeline 35. Due to the presence of the pumps 32 and 34, the user can freely choose to deliver the cleaning liquid and/or steam to the outside as required. As shown in FIG. 2, the pump 32 and the pump 34 of the present embodiment are both provided on the body 11. In other embodiments, a single pump and a plurality of valves can be used in combination to replace the solution of the two pumps in the present embodiment.

In this way, a portion of the cleaning liquid in the cleaning liquid supply tank 1 can be delivered downwards from the cleaning liquid supply tank 1 to the cleaning liquid outlet 111 through the first cleaning liquid output pipeline 31 and applied to the cleaning roller 19. Another portion of the cleaning liquid in the cleaning liquid supply tank 1 is delivered to the steam generator 2 through the second cleaning liquid output pipeline 33 and converted into high-temperature steam. The high-temperature steam is delivered to the steam outlet 115 through the steam output pipeline 35 and applied directly to the surface to be cleaned.

As shown in FIG. 6 again, when the surface cleaning apparatus 10 of the present embodiment works, the cleaning liquid outlet 111 continuously applies the cleaning liquid to the cleaning roller 19 as the cleaning roller 19 rotates around the direction R, and the surface to be cleaned 50 undergoes wet scrubbing during the contacting process of the cleaning roller 19 loaded with the cleaning liquid with the surface to be cleaned 50. After the surface to be cleaned 50 is scrubbed, the used cleaning fluid is contaminated by the dirt on the surface to be cleaned and becomes dirty liquid mixed with the dirt, and the dirty liquid and the solid waste on the surface to be cleaned are removed upwards from the surface to be cleaned at the opening 17 by a centrifugal force generated when the cleaning roller 19 rotates and the suction action of the suction source 4, and conveyed to the roller chamber 18, and then conveyed to the dirty liquid recovery tank 3 through the front suction mouth 15 and the lower suction passage 42 in sequence. The dirty liquid and solid waste will be trapped in the dirty liquid recovery tank 3, and the clean air will flow from the upper suction passage 41 to the suction source 4 and finally escape to the outside. As the cleaning head 12 is pushed to move forwards, the surface 50 subjected to wet cleaning by the cleaning roller 19 will be heated by the steam overflowing from the steam outlet 115. As the temperature of the steam is high, the steam not only heats the surface but also sterilizes the surface with high temperature after being applied to the surface. If there is residual liquid on the surface before the surface is heated by the steam, the residual liquid will be evaporated quickly as the temperature of the surface rises. If there is still residual liquid (or water droplets condensed by steam) on the surface heated by steam, then the residual liquid will be sucked up when the flexible flat suction nozzle 114 passes here and conveyed through the suction bypass 43 to the lower suction passage 42.

After the surface cleaning apparatus in the aforementioned embodiment performs cleaning, the cleaned surface is almost free of the residual liquid. If the surface is the floor, the user may directly walk on the area without waiting. If the surface is a table top, the user may directly place or stack articles on the surface. As high-temperature steam is also used to sterilize the surface to be cleaned while the surface is heated, the surface cleaning apparatus cleans the surface more thoroughly and has a better cleaning effect. Experimental tests show that the higher the temperature of the steam applied to the surface to be cleaned is, the faster the residual liquid on the surface will evaporate. After testing, the surface cleaning apparatus of the invention is able to achieve quick drying in less than 2 minutes on general wooden and tile floors, and even achieve immediate drying in 5 seconds after cleaning by surface cleaning apparatus. In other embodiments, the steam outlet may also be arranged in other positions of the housing, such as the front side or the left and right sides thereof. For example, when the steam outlet is arranged on the front side of the housing, the steam outlet on the front side may soften the dirt on the surface with the steam in advance and at the same time heat the surface, which provides convenience for the subsequent cleaning by the cleaning roller. The heated surface is also more conducive to the evaporation of the residual liquid on the surface after the surface is cleaned by the cleaning roller. In addition, in other embodiments, steam outlets may also be arranged at a plurality of positions including the front side, the rear side, and the left and right sides to meet the requirements of steam application. In addition, the surface cleaning apparatus may also be designed to adopt a horizontal structure or other portable structures.

## Claims

1. A surface cleaning apparatus, comprising:
a suction source (4);
a cleaning head (12) adapted to move along a surface to be cleaned, the head including a housing and a cleaning roller (19), the housing provided with a roller chamber (18) having an opening (17) in a lower portion of the housing and a front suction mouth (15) in fluid communication with the roller chamber, the roller positioned in the roller chamber in such a way as to be able to rotate, at least a portion of the cleaning roller extending through the opening configured to contact the surface to be cleaned, and the front suction mouth in fluid communication with the suction source;
a cleaning liquid supply tank (1) adapted to store cleaning liquid;
a steam generator (2) in fluid communication with the supply tank and configured to heat liquid to generate steam;
a first fluid delivery unit including at least one cleaning liquid outlet (111) for applying the cleaning liquid in the cleaning liquid supply tank to the cleaning roller and/or the surface to be cleaned;
a second fluid delivery unit including at least one steam outlet (115) for applying the steam generated by the steam generator to the surface to be cleaned; and
a dirty liquid recovery tank configured to accept dirty liquid and dirt, the dirty liquid recovery tank being in fluid communication with the front suction mouth;
**characterized in that**
the at least one steam outlet (115) is arranged at the lower portion of the housing and configured to selectively work simultaneously with the at least one fluid outlet (111) so as to heat the surface by overflowing the steam to the surface during the process of cleaning the surface with the cleaning liquid to accelerate the evaporation of residual cleaning liquid on the surface.

2. The surface cleaning apparatus according to claim 1, wherein the at least one steam outlet is configured to spray the steam directly to the surface to be cleaned.

3. The surface cleaning apparatus according to claim 1, wherein the at least one steam outlet is at least partially positioned behind the opening to enable the surface cleaning apparatus to heat a surface cleaned by the cleaning roller with the steam overflowing from the at least one steam outlet.

4. The surface cleaning apparatus according to claim 3, wherein a flexible partition (112) is arranged between the at least one steam outlet and the opening.

5. The surface cleaning apparatus according to claim 3, wherein the cleaning head further comprises a rear suction mouth (1143) provided at a rear portion of the housing, the rear suction mouth is positioned behind the at least one steam outlet, and the rear suction mouth is configured to be in fluid communication with the suction source, wherein the cleaning head comprises a flexible flat suction nozzle (114) arranged at the lower portion of the housing, wherein the flexible flat suction nozzle comprises a pair of flexible scraper lips arranged relatively front and back to each other , and the rear suction mouth is formed at the flexible flat suction nozzle; and at least one of the flexible scraper lips interferes with the surface to be cleaned when the cleaning head moves along the surface.

6. The surface cleaning apparatus according to claim 1, wherein the cleaning head further comprises a flexible liquid-scraping strip positioned behind the at least one steam outlet, and the strip interferes with the surface to be cleaned when the cleaning head moves along the surface to be cleaned.

7. The surface cleaning apparatus according to claim 1, wherein the at least one steam outlet is plural in number.

8. The surface cleaning apparatus according to claim 7, wherein a steam diffuser for diffusing the steam generated by the steam generator is provided at the lower portion of the housing, and the plurality of steam outlets are located at a lower portion of the steam diffuser.

9. The surface cleaning apparatus according to claim 8, wherein the steam diffuser comprises a steam bottom plate (113), and the plurality of steam outlets are arranged on a lower surface of the steam bottom plate.

10. The surface cleaning apparatus according to claim 1, wherein the surface cleaning apparatus further comprises a body (11), the body having a handle (13) at a upper part, and the cleaning head is in articulately connection to a lower part of the body, wherein the dirty liquid recovery tank and the cleaning liquid supply tank are both detachably positioned on the body.

11. The surface cleaning apparatus according to claim 1, wherein the first fluid delivery unit comprises a first pump (32) arranged between the cleaning liquid supply tank and the at least one cleaning liquid outlet.

12. The surface cleaning apparatus according to claim 1, wherein the second fluid delivery unit comprises a second pump (34) arranged between the cleaning liquid supply tank and the steam generator.

13. The surface cleaning apparatus according to claim 1, wherein a cleaning cloth for scrubbing the surface to be cleaned is provided at the lower portion of the housing, the cleaning cloth is made of a steam-permeable material, and the steam outlet is configured to spray the steam to the cleaning cloth.

14. The surface cleaning apparatus according to claim 1, wherein a brush for scrubbing the surface to be cleaned is further provided at the lower portion of the housing.

15. The surface cleaning apparatus according to claim 1, wherein at least part of the steam outlets are arranged on the front side or left and right sides of the opening.

## Patentansprüche

1. Oberflächenreinigungsgerät, umfassend:
eine Saugquelle (4);
einen Reinigungskopf (12), der dazu geeignet ist, sich entlang einer zu reinigenden Oberfläche zu bewegen, wobei der Kopf ein Gehäuse und eine Reinigungswalze (19) umfasst, wobei das Gehäuse mit einer Walzenkammer (18) versehen ist, die eine Öffnung (17) in einem unteren Abschnitt des Gehäuses und eine vordere Saugöffnung (15) aufweist, die fluidisch mit der Walzenkammer verbunden ist, wobei die Walze so in der Walzenkammer angeordnet ist, dass sie drehbar ist, wobei zumindest ein Teil der Reinigungswalze durch die Öffnung hindurchragt, um mit der zu reinigenden Oberfläche in Kontakt zu treten, und wobei die vordere Saugöffnung fluidisch mit der Saugquelle verbunden ist;
einen Reinigungsflüssigkeitsvorratsbehälter (1), der dazu geeignet ist, Reinigungsflüssigkeit zu speichern;
einen Dampferzeuger (2), der fluidisch mit dem Vorratsbehälter verbunden ist und dazu konfiguriert ist, Flüssigkeit zu erhitzen, um Dampf zu erzeugen;
eine erste Fluidzuführeinheit, die mindestens einen Reinigungsflüssigkeitsauslass (111) umfasst, um die Reinigungsflüssigkeit aus dem Reinigungsflüssigkeitsvorratsbehälter auf die Reinigungswalze und/oder auf die zu reinigende Oberfläche aufzutragen;
eine zweite Fluidzuführeinheit, die mindestens einen Dampfauslass (115) umfasst, um den vom Dampferzeuger erzeugten Dampf auf die zu reinigende Oberfläche aufzutragen; und
einen Schmutzwasserauffangbehälter, der dazu konfiguriert ist, Schmutzwasser und Schmutz aufzunehmen, wobei der Schmutzwasserauffangbehälter fluidisch mit der vorderen Saugöffnung verbunden ist;
**dadurch gekennzeichnet, dass**
der mindestens eine Dampfauslass (115) an dem unteren Abschnitt des Gehäuses angeordnet ist und dazu konfiguriert ist, selektiv gleichzeitig mit dem mindestens einen Flüssigkeitsauslass (111) zu arbeiten, um die Oberfläche durch Überströmen des Dampfes auf die Oberfläche während des Reinigens der Oberfläche mit der Reinigungsflüssigkeit zu erhitzen, um die Verdampfung von Restreinigungsflüssigkeit auf der Oberfläche zu beschleunigen.

2. Oberflächenreinigungsgerät nach Anspruch 1, wobei der mindestens eine Dampfauslass dazu konfiguriert ist, den Dampf direkt auf die zu reinigende Oberfläche zu sprühen.

3. Oberflächenreinigungsgerät nach Anspruch 1, wobei der mindestens eine Dampfauslass zumindest teilweise hinter der Öffnung angeordnet ist, um dem Oberflächenreinigungsgerät zu ermöglichen, eine durch die Reinigungswalze gereinigte Oberfläche mit dem aus dem mindestens einen Dampfauslass überströmenden Dampf zu erhitzen.

4. Oberflächenreinigungsgerät nach Anspruch 3, wobei eine flexible Trennwand (112) zwischen dem mindestens einen Dampfauslass und der Öffnung angeordnet ist.

5. Oberflächenreinigungsgerät nach Anspruch 3, wobei der Reinigungskopf ferner eine hintere Saugöffnung (1143) umfasst, die an einem hinteren Abschnitt des Gehäuses angeordnet ist, wobei die hintere Saugöffnung hinter dem mindestens einen Dampfauslass angeordnet ist und die hintere Saugöffnung dazu konfiguriert ist, fluidisch mit der Saugquelle verbunden zu sein, wobei der Reinigungskopf eine flexible Flachsaugdüse (114) umfasst, die an dem unteren Abschnitt des Gehäuses angeordnet ist, wobei die flexible Flachsaugdüse ein Paar flexibler Abstreiflappen umfasst, die einander relativ vorne und hinten zugeordnet sind, und wobei die hintere Saugöffnung an der flexiblen Flachsaugdüse ausgebildet ist; und mindestens einer der flexiblen Abstreiflappen mit der zu reinigenden Oberfläche interferiert, wenn sich der Reinigungskopf entlang der Oberfläche bewegt.

6. Oberflächenreinigungsgerät nach Anspruch 1, wobei der Reinigungskopf ferner einen flexiblen Flüssigkeitsabstreifstreifen umfasst, der hinter dem mindestens einen Dampfauslass angeordnet ist, und wobei der Streifen mit der zu reinigenden Oberfläche interferiert, wenn sich der Reinigungskopf entlang der zu reinigenden Oberfläche bewegt.

7. Oberflächenreinigungsgerät nach Anspruch 1, wobei der mindestens eine Dampfauslass mehrfach vorhanden ist.

8. Oberflächenreinigungsgerät nach Anspruch 7, wobei ein Dampfdiffusor zum Diffundieren des vom Dampferzeuger erzeugten Dampfes an dem unteren Abschnitt des Gehäuses vorgesehen ist, und wobei die Mehrzahl der Dampfauslässe an einem unteren Abschnitt des Dampfdiffusors angeordnet sind.

9. Oberflächenreinigungsgerät nach Anspruch 8, wobei der Dampfdiffusor eine Dampfbodenplatte (113) umfasst und die Mehrzahl der Dampfauslässe auf einer Unterseite der Dampfbodenplatte angeordnet sind.

10. Oberflächenreinigungsgerät nach Anspruch 1, wobei das Oberflächenreinigungsgerät ferner ein Gehäuse (11) umfasst, das an einem oberen Abschnitt einen Griff (13) aufweist, und der Reinigungskopf gelenkig mit einem unteren Abschnitt des Gehäuses verbunden ist, wobei der Schmutzwasserauffangbehälter und der Reinigungsflüssigkeitsvorratsbehälter beide lösbar an dem Gehäuse angeordnet sind.

11. Oberflächenreinigungsgerät nach Anspruch 1, wobei die erste Fluidzuführeinheit eine erste Pumpe (32) umfasst, die zwischen dem Reinigungsflüssigkeitsvorratsbehälter und dem mindestens einen Reinigungsflüssigkeitsauslass angeordnet ist.

12. Oberflächenreinigungsgerät nach Anspruch 1, wobei die zweite Fluidzuführeinheit eine zweite Pumpe (34) umfasst, die zwischen dem Reinigungsflüssigkeitsvorratsbehälter und dem Dampferzeuger angeordnet ist.

13. Oberflächenreinigungsgerät nach Anspruch 1, wobei ein Reinigungstuch zum Abreiben der zu reinigenden Oberfläche an dem unteren Abschnitt des Gehäuses vorgesehen ist, wobei das Reinigungstuch aus dampfdurchlässigem Material besteht und der Dampfauslass dazu konfiguriert ist, den Dampf auf das Reinigungstuch zu sprühen.

14. Oberflächenreinigungsgerät nach Anspruch 1, wobei an dem unteren Abschnitt des Gehäuses ferner eine Bürste zum Abreiben der zu reinigenden Oberfläche vorgesehen ist.

15. Oberflächenreinigungsgerät nach Anspruch 1, wobei zumindest ein Teil der Dampfauslässe an der Vorderseite oder den linken und rechten Seiten der Öffnung angeordnet ist.

## Revendications

1. Appareil de nettoyage de surface, comprenant :
une source d'aspiration (4) ;
une tête de nettoyage (12) conçue pour se déplacer le long d'une surface à nettoyer, la tête comprenant un boîtier et un rouleau de nettoyage (19), le boîtier étant pourvu d'une chambre de rouleau (18) ayant une ouverture (17) dans une partie inférieure du boîtier et une bouche d'aspiration avant (15) en communication fluidique avec la chambre de rouleau, le rouleau étant positionné dans la chambre de rouleau de manière à pouvoir tourner, au moins une partie du rouleau de nettoyage s'étendant à travers l'ouverture de manière à entrer en contact avec la surface à nettoyer, et la bouche d'aspiration avant étant en communication fluidique avec la source d'aspiration ;
un réservoir de liquide de nettoyage (1) conçu pour stocker un liquide de nettoyage ;
un générateur de vapeur (2) en communication fluidique avec le réservoir et conçu pour chauffer le liquide afin de générer de la vapeur ;
une première unité de distribution de fluide comprenant au moins un orifice de sortie de liquide de nettoyage (111) pour appliquer le liquide de nettoyage du réservoir sur le rouleau de nettoyage et/ou la surface à nettoyer ;
une seconde unité de distribution de fluide comprenant au moins un orifice de sortie de vapeur (115) pour appliquer la vapeur générée par le générateur de vapeur sur la surface à nettoyer ; et
un réservoir de récupération de liquide sale conçu pour recueillir le liquide sale et les saletés, le réservoir de récupération de liquide sale étant en communication fluidique avec la bouche d'aspiration avant ;
**caractérisé en ce que**
le ou les orifices de sortie de vapeur (115) sont disposés au niveau de la partie inférieure du boîtier et configurés pour fonctionner sélectivement simultanément avec le ou les orifices de sortie de liquide (111) de manière à chauffer la surface par débordement de la vapeur vers la surface pendant le processus de nettoyage de la surface avec le liquide de nettoyage afin d'accélérer l'évaporation du liquide de nettoyage résiduel sur la surface.

2. L'appareil de nettoyage de surface selon la revendication 1, dans lequel le ou les orifices de sortie de vapeur sont configurés pour pulvériser directement la vapeur sur la surface à nettoyer.

3. L'appareil de nettoyage de surface selon la revendication 1, dans lequel le ou les orifices de sortie de vapeur sont au moins partiellement positionnés derrière l'ouverture de manière à permettre à l'appareil de nettoyage de surface de chauffer une surface nettoyée par le rouleau de nettoyage à l'aide de la vapeur débordant du ou des orifices de sortie de vapeur.

4. L'appareil de nettoyage de surface selon la revendication 3, dans lequel une cloison flexible (112) est disposée entre le ou les orifices de sortie de vapeur et l'ouverture.

5. L'appareil de nettoyage de surface selon la revendication 3, dans lequel la tête de nettoyage comprend en outre une bouche d'aspiration arrière (1143) disposée dans une partie arrière du boîtier, la bouche d'aspiration arrière étant positionnée derrière le ou les orifices de sortie de vapeur, et la bouche d'aspiration arrière étant configurée pour être en communication fluidique avec la source d'aspiration, dans lequel la tête de nettoyage comprend une buse d'aspiration plate et flexible (114) disposée au niveau de la partie inférieure du boîtier, la buse d'aspiration plate et flexible comprenant une paire de lèvres de raclage flexibles disposées l'une à l'avant et l'autre à l'arrière, et la bouche d'aspiration arrière étant formée au niveau de la buse d'aspiration plate et flexible ; et au moins l'une des lèvres de raclage flexibles interfère avec la surface à nettoyer lorsque la tête de nettoyage se déplace le long de ladite surface.

6. L'appareil de nettoyage de surface selon la revendication 1, dans lequel la tête de nettoyage comprend en outre une bande de raclage de liquide flexible positionnée derrière le ou les orifices de sortie de vapeur, ladite bande interférant avec la surface à nettoyer lorsque la tête de nettoyage se déplace le long de ladite surface.

7. L'appareil de nettoyage de surface selon la revendication 1, dans lequel le nombre d'orifices de sortie de vapeur est multiple.

8. L'appareil de nettoyage de surface selon la revendication 7, dans lequel un diffuseur de vapeur destiné à diffuser la vapeur générée par le générateur de vapeur est prévu au niveau de la partie inférieure du boîtier, et la pluralité d'orifices de sortie de vapeur est située au niveau d'une partie inférieure du diffuseur de vapeur.

9. L'appareil de nettoyage de surface selon la revendication 8, dans lequel le diffuseur de vapeur comprend une plaque inférieure de vapeur (113), et la pluralité d'orifices de sortie de vapeur est disposée sur une surface inférieure de la plaque inférieure de vapeur.

10. L'appareil de nettoyage de surface selon la revendication 1, dans lequel l'appareil de nettoyage de surface comprend en outre un corps (11), le corps comportant une poignée (13) dans une partie supérieure, et la tête de nettoyage étant reliée de manière articulée à une partie inférieure du corps, dans lequel le réservoir de récupération de liquide sale et le réservoir de liquide de nettoyage sont tous deux positionnés de manière amovible sur le corps.

11. L'appareil de nettoyage de surface selon la revendication 1, dans lequel la première unité de distribution de fluide comprend une première pompe (32) disposée entre le réservoir de liquide de nettoyage et le ou les orifices de sortie de liquide de nettoyage.

12. L'appareil de nettoyage de surface selon la revendication 1, dans lequel la seconde unité de distribution de fluide comprend une seconde pompe (34) disposée entre le réservoir de liquide de nettoyage et le générateur de vapeur.

13. L'appareil de nettoyage de surface selon la revendication 1, dans lequel un chiffon de nettoyage destiné à frotter la surface à nettoyer est prévu au niveau de la partie inférieure du boîtier, le chiffon de nettoyage étant constitué d'un matériau perméable à la vapeur, et l'orifice de sortie de vapeur étant configuré pour pulvériser la vapeur sur le chiffon de nettoyage.

14. L'appareil de nettoyage de surface selon la revendication 1, dans lequel une brosse destinée à frotter la surface à nettoyer est en outre prévue au niveau de la partie inférieure du boîtier.

15. L'appareil de nettoyage de surface selon la revendication 1, dans lequel au moins une partie des orifices de sortie de vapeur est disposée sur le côté avant ou sur les côtés gauche et droit de l'ouverture.
